# EUROPÄISCHE PATENTSCHRIFT

(11) **EP 1 581 469 B1**
(45) Veröffentlichungstag und Bekanntmachung des Hinweises auf die Patenterteilung: **21.10.2015**
(21) Anmeldenummer: 03769471.8
(22) Anmeldetag: 30.10.2003
(51) Int. Cl.: C07C 41/06, C07C 41/03, C07C 43/225, C07C 41/01, C07C 51/15, C07C 63/04, C07D 339/06, C07D 339/08

(54) **VERFAHREN ZUR HERSTELLUNG VON 2,6-SUBSTITUIERTEN TRANS DEKALINDERIVATEN**
METHOD FOR PRODUCING 2,6-SUBSTITUTED TRANS DECALIN DERIVATIVES
PRODUCTION DE DÉRIVÉS DE 2,6-SUBSTITUÉS DE LA DÉCALINE

(30) Priorität: 03.12.2002 DE 10256362
(43) Veröffentlichungstag der Anmeldung: 05.10.2005
(73) Patentinhaber: Merck Patent GmbH, 64293 Darmstadt (DE)
(72) Erfinder: POETSCH, Eike, 64367 Mühltal (DE); BINDER, Werner, 64807 Dieburg (DE); KIRSCH, Peer, 64342 Seeheim-Jugenheim (DE); TAUGERBECK, Andreas, 64285 Darmstadt (DE)
(86) Internationale Anmeldenummer: PCT/EP2003/012039
(87) Internationale Veröffentlichungsnummer: WO 2004/050594

(56) Entgegenhaltungen:
- EP-A- 1 108 700
- DE-A- 10 010 537
- JP-A- 2001 002 597
- L.F. FIESER ET AL.: "The synthesis of 3-substituted derivatives of methylcholanthrene" JOURNAL OF THE AMERICAN CHEMICAL SOCIETY, Bd. 59, 1937, Seiten 2561-2564, XP002267305

## Beschreibung

Die vorliegende Erfindung betrifft ein Verfahren zur Herstellung von flüssigkristallinen Verbindungen, die von hydrierten Derivaten des Naphthalins abgeleitet sind, und die als Charakteristikum eine -CF₂O- Brücke im Molekül aufweisen. Das Verfahren geht dabei von halogensubstituierten Naphthalinderivaten aus, die nach Überführung in die entsprechenden Säuren über den Umweg der Grignard-Verbindungen in die Zielmoleküle überführt werden. Vorzugsweise geschieht dies über die Dithioorthoester.

Flüssigkristalle haben ein breites Anwendungsfeld gefunden, seitdem vor etwa 30 Jahren die ersten kommerziell anwendbaren flüssigkristallinen Verbindungen aufgefunden wurden. Bekannte Anwendungsgebiete sind beispielsweise Anzeigedisplays für Uhren, Taschenrechner und Telefone. Weitere Anwendungsgebiete sind Displays von tragbaren Computern und Navigationssystemen sowie Videoapplikationen und PC-Monitore. Insbesondere für die zuletzt genannten Anwendungen werden hohe Anforderungen an Schaltzeiten und den Kontrast der Abbildungen gestellt.

Um für kommerzielle Anwendungen brauchbar zu sein, müssen die flüssigkristallinen Moleküle bestimmte Eigenschaften aufweisen. Um Geräte mit einem Flüssigkristalldisplay unter unterschiedlichen Klimabedingungen einsetzen zu können, müssen die Moleküle über einen möglichst großen Temperaturbereich, der im Bereich der Raumtemperatur liegt, eine stabile nematische Phase ausbilden. Die Verbindungen müssen also einen niedrigen Schmelzpunkt und einen hohen Klärpunkt besitzen.

Um niedrige Schaltzeiten verwirklichen zu können, müssen die Moleküle eine niedrige Rotationsviskosität aufweisen. So werden für Videoapplikationen Schaltzeiten von weniger als 16,7 Millisekunden gefordert. Ferner sollen die flüssigkristallinen Moleküle eine hohe dielektrische Anisotropie aufweisen, so dass nur niedrige Schwellenspannungen erforderlich sind. Dies bedeutet einen niedrigen Energiebedarf, so dass beispielsweise in Laptops kleinere und damit leichtere Akkus verwendet werden können. Für die Gestaltung des Displays sind ferner die Doppelbrechungseigenschaften der Moleküle von Bedeutung, die den Kontrast und den nutzbaren Blickwinkel beeinflussen.

Um all diese Anforderungen gleichzeitig erfüllen zu können, werden keine reinen Substanzen verwendet, sondern Gemische, die meist 5 bis 15 unterschiedliche Komponenten umfassen. Dies bedeutet, dass die einzelnen Komponenten miteinander verträglich sein müssen, also sich beispielsweise ausreichend ineinander lösen.

Für moderne Aktivmatrixdisplays wird ein hoher Kontrast der Abbildungen gewünscht. Die flüssigkristallinen Verbindungen müssen daher einen hohen spezifischen Widerstand und eine hohe voltage holding ratio aufweisen.

Als flüssigkristalline Verbindungen mit einem besonders hohen spezifischen Widerstand haben sich die Verbindungen erwiesen, die fluorhaltige Gruppen in ihrem Molekülgerüst enthalten. So werden zum Beispiel in der EP 0 844 229 A1 flüssigkristalline Verbindungen beschrieben, die eine CF₂O-Brück enthalten. Zur Herstellung dieser Brücke werden verschiedene Verfahren vorgeschlagen. Nach einem der beschriebenen Verfahren wird zunächst ein aromatisches Halogenid in eine Grignard-Verbindung oder in eine lithiierte Verbindung überführt und dann mit Schwefelkohlenstoff in die Dithiocarbonsäure überführt. Die Dithiocarbonsäure wird mit einem Phenol in Gegenwart eines Alkalimetallhydrids und Jod in einen Thioester überführt. Mit einem Fluorierungsmittel wird dann die gewünschte CF₂O-Brücke gebildet.

Nach einem anderen Verfahren wird vorgeschlagen, zunächst ein Cyclohexanon mit Tris(dimethylamino)phosphan und Dibromdifluormethan umzusetzen, um ein Difluormethylenhexyliden zu erhalten. An dieses wird zunächst Brom addiert und dann durch Reaktion mit einem Phenolat unter gleichzeitiger Abspaltung von Bromwasserstoff unter Ausbildung einer -CF₂-O-Brücke verethert.

Flüssigkristalline Naphthaline, Tetraline und Dekaline sind seit längerem bekannt (M. Petrzilka, K. Schleich, Helv. Chim. Acta 65,1982, Seiten 1242 ff., H. Zollinger et al., Helv. Chem. Acta 64, 1981, Seiten 1847 ff., und ibid 66, 1983, Seiten 1574 ff., E. Poetsch, Kontakte 2, 1988, Seiten 15 ff.). Die JP 2001-002597 offenbart Dekaline mit einer -CF₂O- Gruppe als Flüssigkristallines Material ohne eine Synthesevorschrift.

Ihre Verwendung in Flüssigkristallanzeigen ist bisher trotz der breiteren Mesophasen (W. Schäfer, H. Altmann, H. Zaschke, H.H. Deutscher, Mol. Cryst. Liq. Cryst. 95, 1983, Seiten 63 ff.), in Vergleich zu den (kommerziell benutzten) Cyclohexyl- und Phenylring-haltigen Verbindungen, unterblieben, offenbar deshalb, weil durch den erhöhten sterischen Anspruch der Naphthalingerüste eine höhere Fließ- und Rotationsviskosität resultiert, die zu unerwünschten, verlängerten Schaltzeiten führt.

Flüssigkristalline Verbindungen, die sich von Naphthalinderivaten bzw. dessen (teil)hydrierten Derivaten ableiten und eine CF₂O-Brücke aufweisen, werden in der DE 40 06921 A1, JP 2000-1116370/10, JP 1133495, WO 00/10952 A1, JP 2001-19649 und JP 2000-355557 beschrieben. Aus den genannten Dokumenten geht zum einen jedoch kein Hinweis hervor, dass die beschriebenen Verbindungen nicht die üblichen, vorstehend dargestellten Nachteile von flüssigkristallinen Naphthalinderivaten aufweisen. Zum anderen offenbaren die genannten Dokumente keine technisch durchführbare Synthese zur Herstellung von Naphthalinderivaten mit CF₂O-Brücken.

Aufgabe der vorliegenden Erfindung ist daher die Bereitstellung eines solchen Verfahrens. Diese Aufgabe wird gelöst durch ein Verfahren zur Herstellung einer Verbindung der allgemeinen Formel

R-(A¹-Z-)ₘ B-CF₂O-A²-(A³)ₙ-R' (I)

in der
- R: Alkyl mit 1 bis 12 C-Atomen, vorzugsweise mit 1 bis 5 C-Atomen und besonders bevorzugt mit 1, 3 und 5 C-Atomen, in dem eine oder mehrere CH₂-Gruppen unabhängig voneinander durch O, CF₂, CH=CH, CH=CF, CF=CF ersetzt sein können mit der Maßgabe, dass Peroxidstrukturen O-O und Formaldehydacetale O-CH₂-O ausgeschlossen sind,
- A¹: unabhängig voneinander 1,4-Cyclohexylen, 2,5-1,3-Dioxanylen, 1,3-Cyclobutylen oder 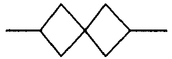
- A², A³: 1,4-Phenylen, in denen unabhängig voneinander ein bis vier Wasserstoffe durch Fluor oder ein oder zwei CH-Gruppen durch N ersetzt sein können,
- Z: Einfachbindung, -CH₂-CH₂-, -CF₂-CF₂-, -CH=CH-, -CF=CF-, -CH=CF- oder -CF=CH-,
- B: 2,6-disubstituiertes trans-Dekalin,
- R': R, F, OCF₃, OCF₂H, CF₃, Cl, SF₅, CN, NCS, und
- m, n: 0,
bedeuten,
umfassend die folgenden Schritte:
a) Überführen einer Verbindung der allgemeinen Formel

   R-(A¹-Z-)ₘBX (II),

   in der X ein Halogen bedeutet und die anderen Symbole die in Bezug auf Formel (I) angegebene Bedeutung haben, in die entsprechende Grignard-Verbindung, Umsetzen der erhaltenen Grignard-Verbindung mit CO₂ und Hydrolyse zur entsprechenden Carbonsäure der Formel

   R-(A¹-Z-)ₘB-CO₂H (IV)
b) Überführung der Carbonsäure der Formel (IV) in das entsprechende Bisalkylthionium-Salz und Umsetzung dieses Salzes mit einem Phenol der allgemeinen Formel

   HO-A²(-A³)ₙ-R' (III),

   in der A², A³, R' und n die in Bezug auf Formel (I) angegebene Bedeutung haben, unter wasserabspaltenden Bedingungen und unter Erhalt der entsprechenden Ester, und
c) Umsetzung der erhaltenen Ester in einer oxidativen Fluordesulfurierung in die entsprechenden Ether der allgemeinen Formel (I).

In einer bevorzugten Ausführungsform der vorliegenden Erfindung ist X in Formel (II) ausgewählt aus der Gruppe CI, Br, I, besonders bevorzugt ist X = Br.

Die Halogen-substituierten Verbindungen der Formel (II) sind auf an sich bekannte Weise zugänglich. Vorzugsweise werden sie aus den entsprechenden Alkoholen hergestellt, durch Umsetzung mit dem entsprechenden Halogenwasserstoff, Thionylhalogeniden oder mittels Halogen/PPh₃. Die Überführung der Verbindungen der Formel (II) in die entsprechende Grignard-Verbindung ist auf die in der DE 102 20 549 A1 beschriebene Art, die sich auf die Herstellung von Dekalinderivate bezieht, möglich. Die erhaltenen Grignard-Verbindungen der Dekalinderivate weisen wegen ihrer konfigurativen Instabilität eine all trans-Konfiguration auf, d.h. sowohl ein axiales wie auch ein äquatoriales Halogenid liefert ein äquatoriales MgBr-Derivat. Diese Stereochemie bleibt auch in den nachfolgenden Säuren (IV) bzw. den daraus gebildeten Umsetzungsprodukten erhalten. Das in der DE 102 20 549 A1 offenbarte Verfahren zur Herstellung von Grignard-Verbindungen ist ein integraler Bestandteil der vorliegenden Anmeldung und durch Bezugnahme eingeschlossen.

Das Verfahren zur Herstellung der Grignard-Verbindungen wird nachfolgend nochmals kurz dargelegt.

Hierzu wird eine Verbindung der Formel (II) mit B = 2,6-Dekalinyl, und X = Halogen mit Magnesium in einem Lösungsmittel, das mindestens ein unpolares Lösungsmittel und mindestens ein polares Lösungsmittel aufweist, unter externer Wärmezufuhr umgesetzt.

Es ist von besonderem Vorteil, dass die gewünschte Grignard-Reaktion gemäß der Erfindung ohne oder nur mit einem geringen Anteil an beta-Eliminierung, die beispielsweise zu HBr oder HMgBr-Bildung führt, verläuft.

Beispiele für geeignete unpolare Lösungsmittel sind aliphatische und aromatische Kohlenwasserstoffe ohne polare Gruppen, beispielsweise Hexan, Cyclohexan, Benzol, Toluol oder Xylol oder Gemische dieser Lösungsmittel.

Geeignete polare Lösungsmittel sind beispielsweise Ether, wie etwa Diethylether, Methyl-tert-butylether, Dimethoxyethan, Tetrahydrofuran oder Dioxan.

Das Mischungsverhältnis (bezogen auf Volumina) des unpolaren Lösungsmittels oder Gemischs zu dem polaren Lösungsmittel oder -gemisch beträgt in der Regel 10 : 1 bis 1 : 2, vorzugsweise von 8 : 1 bis 1 : 1 und besonders bevorzugt 6 : 1 bis 2 : 1.

Ein besonders bevorzugtes Lösungsmittelgemisch ist Benzol und/oder Toluol mit Tetrahydrofuran. Hierbei wird vorteilhafterweise ein Mischungsverhältnis von 5 : 1 bis 3 : 1 gewählt.

Neben Lösungsmittelgemischen mit zwei Komponenten sind auch solche mit 3, 4, 5 oder mehr Komponenten einsetzbar, sofern jeweils mindestens ein ausreichend polares und jeweils mindestens ein im wesentlichen unpolares Lösungsmittel vorhanden ist.

Die Umsetzung erfolgt vorteilhafterweise bei Siedehitze des Lösungsmittelgemisches unter Normaldruck. Ein bevorzugter Temperaturbereich liegt bei 40 bis 100°C und besonders bevorzugt bei 50 bis 80°C.

Gemäß einer bevorzugten Ausführungsform wird Magnesium in geeigneter Form, beispielsweise in Form von Spänen, in einer Schutzgasatmosphäre, beispielsweise Stickstoff, vorgelegt, und ein Teil der Lösung, die die Halogenverbindung der Formel (II) enthält, zugegeben. Die so vorgelegte Menge wird erfindungsgemäß mit dem Magnesium erwärmt und ein geeigneter Starter, beispielsweise Jod oder eine geringe Menge an Dibromethan, zugegeben. Nach Anspringen der Reaktion wird über einen Zeitraum, der bis zu 120 Minuten betragen kann, die Hauptmenge der Lösung zugegeben und das Reaktionsgemisch weitere 5 bis 360 Minuten erwärmt, vorzugsweise unter Rückfluss. Erst nach vollendeter Zugabe und andauernder externer Wärmezufuhr reagiert der größte Teil des Magnesiums ab, wobei die dazu notwendige Zeit der externen Wärmezufuhr in der Regel länger ist als die vorherige Zugabezeit der Lösung. Die externe Wärmezufuhr ist erfindungsgemäß vorgesehen, um die Reaktion aufrechtzuerhalten, da die Bildung der Grignard-Verbindung nicht ausreichend exotherm ist.

Nach Abkühlen wird die Grignard-Verbindung auf dem Fachmann bekannte Weise abgetrennt und aufgereinigt, vorzugsweise wird die nachfolgende Reaktion unter Verwendung der erhaltenen Reaktionslösung durchgeführt. Anschließend wird die erhaltene Grignard-Verbindung mit CO₂ umgesetzt. Dabei bleibt die Stereochemie des Moleküls erhalten (vergleiche Schema I).

Die Darstellung von Dekalincarbonsäurederivaten wird generell ohne vorherige Isolation der Grignard-Verbindung durchgeführt, die Grignard-Verbindung wird also in situ erzeugt und direkt mit CO₂ umgesetzt. Es kommen somit die gleichen Lösungsmittel zum Einsatz, die oben in Zusammenhang mit der Herstellung der Grignard-Verbindung erwähnt wurden.

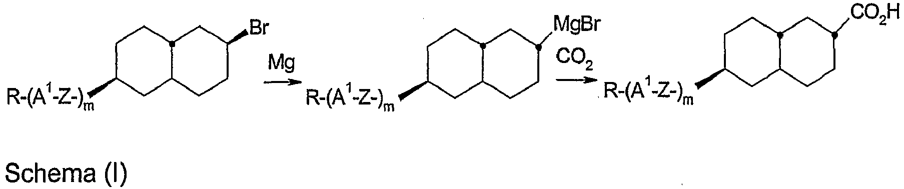

Um eine Zersetzung des Grignardreagenzes durch eine Zerewitinoff-Reaktion, hervorgerufen durch eine Kondenswasserkontamination auf der Oberfläche des Trockeneises, zu vermeiden, wird vorzugsweise gasförmiges CO₂ eingesetzt.

Nach Beendigung der Reaktion wird durch Hydrolyse die Carbonsäure freigesetzt. Die Carbonsäure bzw. das Salz wird dann auf an sich bekannte Weise isoliert.

Aus der Carbonsäure wird anschließend die Zielverbindung, die eine -CF₂O-Brücke enthält, gebildet. Dazu existieren mehrere Möglichkeiten, von denen einige über die entsprechenden Ester verlaufen.

In einer Ausführungsform werden aus den Säuren (IV) und den Phenolen der Formel (III) HO-A²(-A³)ₙ-R', in der A², A³, R' und n die in Bezug auf Formel (I) angegebene Bedeutung haben, die Ester gebildet, wobei die Edukte unter wasserabspaltenden Bedingungen miteinander zur Reaktion gebracht werden. In vielen Fällen wird eine wasserabspaltende Substanz benutzt, z.B. Cyclohexylcarbodiimid oder das mittels eines gemischten anorganischen Säureanhydrids (SOCl₂, PCl₃, POCl₃, PBr₃) dargestellte Säurehalogenid in Gegenwart einer Base (Pyridin, 4-Dimethylaminopyridin, Triethylamin) zum entsprechenden Ester umgesetzt.

Vorzugsweise wird die Umsetzung in Gegenwart von Cyclohexylcarbodiimid durchgeführt, unter dem Fachmann bekannten Bedingungen.

Im Anschluß an die Herstellung der Ester werden diese unter an sich bekannten Bedingungen zu den gewünschten Verbindungen der Formel (I) umgesetzt.

Bevorzugte Methoden umfassen die Schwefelung mit Lawesson-Reagenz (2,4-Bis(4-Methoxyphenyl)-1,2,3,4-dithiadiphosphetan-2,4-dithion) unter Bildung der Thioester als Zwischenprodukt und einer nachfolgenden oxidativen Fluordesulfurierung wobei vorzugsweise ein Bromierungsmittel zur Desulfurierung eingesetzt wird.

Beispiele für geeignete Fluorierungsmittel umfassen aliphatische und aromatische Amin-Fluorwasserstoff-Komplexe, Pyridin-Fluorwasserstoffkomplexe, NEt₃•3HF, 50 % HF in Pyridin, Melamin•HF und Polyvinylpyridin•HF.

Beispiele für geeignete Oxidationsmittel/Bromierungsmittel umfassen Verbindungen, die Haloniumäquivalente freisetzen, vorzugsweise aus der Gruppe Dibromhydantoin, Dimethyldibromhydantoin, N-Bromsuccinimid, N-Iodsuccinimid, 1,3-Dibrom-5,5-Dimethyfhydantoin, SO₂Cl₂, SO₂ClF, Nitrosonium- und Nitroniumsalze, Chloramin T und Brom, besonders bevorzugt Brom.

Für die vorstehend genannten Herstellungsverfahren wird beispielsweise verwiesen auf T. Hiyama et al., Bull. Chem. Soc. Jpn. 73, 2000, 1875; und Tetrahedron Letters 33, 1992, 4173.

Die am meisten bevorzugte Methode zur Herstellung der Ether (I) aus den Säuren (IV) ist die Überführung in die Bis(alkylthio)carbenium-Salze (V) und deren Überführung in die gewünschten Verbindungen der Formel (I), durch Umsetzung mit den Phenolen der Formel (III) in Gegenwart eines Fluorierungsmittels und eines Oxidationsmittels.

Das Verfahren ist in dem nachfolgenden Schema (II) dargestellt.

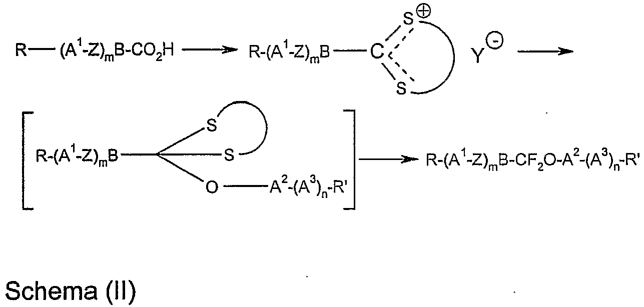

Dabei wird die Carbonsäure (IV) mit einem Alkylthiol zum Bis(alkylthio)-carbenium-Salz (V) umgesetzt. An Stelle einer Carbonsäure kann auch ein Carbonsäurederivat, in dem an Stelle der-OH-Gruppe Halogen, Pseudohalogen, substituiertes Sulfonat, Alkoxy oder Phenoxy vorhanden ist, oder ein Anhydrid eingesetzt werden. Dies ist jedoch nicht bevorzugt, da dies einen weiteren Reaktionsschritt umfassen würde.

Vorzugsweise werden Thiole verwendet, die zur Ausbildung eines cyclischen Kations führen. Insbesondere geeignet sind Ethandithiol, Propandithiol und 1,2-Benzoldithiol, die zur Ausbildung von Dithianylium- bzw. Dithiolanyliumsalzen führen. Das Salz (V) wird anschließend mit einem Phenol (III) zum Orthoester (VI) umgesetzt. Dieser wird im allgemeinen nicht isoliert, sondern direkt oxidativ fluorierend zum Ether (I) umgesetzt. Das vorstehend beschriebene Verfahren zur Herstellung der Ether (I) wird in der WO 01/64667 beschrieben. Dieses Verfahren ist ein integraler Bestandteil der vorliegenden Anmeldung und durch Bezugnahme in diese eingeschlossen.

In einer alternativen, hier nicht beanspruchten Ausführungsform wird das Bisalkylthionium-Salz ausgehend von einer Keto-Verbindung der Formel (II), in der X eine =O-Gruppierung und B ein Dekalinylrest ist, erhalten. Diese Herstellung geschieht auf an sich bekannte Weise. Beispielhaft sei hier auf D.J. Ager, Org. React. 38, 1990, Seiten 1 bis 223, insbesondere die Seiten 63, 95 und 96, verwiesen. Dabei werden Ketone zu dem deprotonierten 2-Silyl-(1,3)-dithian gegeben und nach Erwärmen über 15 bis 90 Minuten auf Zimmertemperatur gegebenenfalls weitere 90 Minuten bei dieser Temperatur gehalten. Nach üblicher Aufarbeitung unter Zugabe von NH₄Cl-Lösung werden so Ketendithioketale erhalten, die nachfolgend in die Bisalkylthionium-Salze überführt werden. Dies geschieht im allgemeinen durch Ansäuern. Danach wird das erhaltene Salz in Gegenwart eines Fluorierungs- und Oxidationsmittels mit dem Phenol (III) zur Verbindung (I) umgesetzt. Ein bevorzugtes Verfahren zur Herstellung der Ketendithioketale ist die Umsetzung mit einem 2-Silyl-1,3-Dithian, das gegebenenfalls substituiert sein kann. Besonders bevorzugt ist der Einsatz von 2-Trimethylsilyl-1,3-dithian. Die Umsetzung erfolgt vorzugsweise in Gegenwart einer deprotonierenden Verbindung, beispielsweise Alkyllithium, vorzugsweise n-Butyllithium. Vorzugsweise liegt die Reaktionstemperatur bei Werten von -130 bis 0°C, besonders bevorzugt -35 bis 0°C. Bevorzugte Lösungsmittel sind ausgewählt aus der Gruppe der Ether und Halogenalkane, beispielsweise Diethylether, Tetrahydrofuran oder Dichlormethan bzw. Gemische daraus.

Das Verfahren der Herstellung von Bis(alkylthio)carbeniumsalzen aus Ketonen und die Umsetzung zu Verbindungen mit CF₂O-Brücke ist in der DE 101 05313 A1 beschrieben. Der sich auf dieses Verfahren beziehende Teil der DE 101 05313 A1 ist ein integraler Bestandteil der vorliegenden Erfindung und durch Bezugnahme eingeschlossen.

Die Säure, die zur Protonierung des Ketendithioketals eingesetzt wird, ist eine der allgemeinen Formel H⁺Y⁻, wobei Y⁻ein nicht- oder schwach-koordinierendes Anion ist. Vorzugsweise ist Y⁻ ausgewählt aus der Gruppe Halogenide, Tetrafluoroborat, Hexafluorophosphat, Perchlorat, Alkylcarbonat, Arylcarbonat, Alkylsulfonat und Arylsulfonat. Dabei können in den Alkyl- und Arylgruppen, ein, mehrere oder alle H-Atome durch Fluor oder Chlor substituiert sein. Besonders bevorzugte Säuren sind Trifluormethansulfonsäure und Tetrafluorborsäure-Diethyletherkomplex.

Die Säure wird in etwa äquimolarer Menge bezogen auf die umzusetzenden Ketendithioketal-Einheiten eingesetzt. Die Umsetzung erfolgt vorteilhafterweise bei einer Temperatur in einem Bereich von -80 bis +30°C in einem inerten polaren Lösungsmittel oder Lösungsmittelgemisch. Geeignete Lösungsmittel sind beispielsweise Ether und Halogenalkane und Gemische daraus, beispielsweise Diethylether, Tetrahydrofuran oder Dichlormethan.

Das Bis(alkylthio)carbeniumsalz weist vorzugsweise ein nicht- oder schwach-koordinierendes Anion auf, das besonders bevorzugt ausgewählt ist aus der Gruppe, die gebildet ist von Tetrafluoroborat, Hexafluorophosphat, Perchlorat sowie Perfluoralkylsulfonat, insbesondere Trifluormethansulfonat. Diese Salze sind einfach zu verarbeiten, da sie kaum hygroskopisch sind.

Bei der Umsetzung der Bis(alkylthio)carbeniumsalze mit den Phenolen (III) können als Oxidationsmittel übliche Oxidationsmittel verwendet werden. Bevorzugt wird als Oxidationsmittel eine Verbindung eingesetzt, die Haloniumäquivalente freisetzt. Beispielhafte Oxidationsmittel sind Dimethyldibromhydantoin, N-Bromsuccinimid, N-Jodsuccinimid, 1,3-Dibrom-5,5-Dimethylhydantoin und Brom. Besonders bevorzugt ist Brom, da sich die entstehenden Bromide leicht abtrennen lassen. Ebenfalls geeignet sind beispielsweise SO₂Cl₂, SO₂ClF, Nitrosonium- und Nitroniumsalze sowie Chloramin T.

Als Fluorierungsmittel können übliche Fluorierungsmittel eingesetzt werden. Besonders bevorzugt wird das Fluorierungsmittel ausgewählt aus der Gruppe die gebildet ist von aliphatischen und aromatischen Amin-Fluorwasserstoff-Komplexen, Pyridin-Fluorwasserstoffkomplexen, NEt₃•3HF, 50 % HF in Pyridin, Melamin•HF und Polyvinylpyridin•HF.

Die Erfindung wird in den nachfolgenden, nicht beschränkenden Beispielen erläutert.

### Beispiel 1

### Herstellung von 6β-Propyl-(4aα, 8aβ)-decahydronaphthalin-2α-carbonsäure.

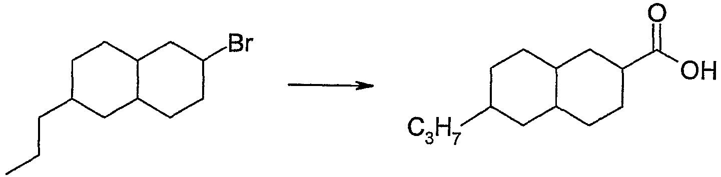

10,0 g (0,4111 mol) Magnesium-Späne wurden vorgelegt und anschließend eine Lösung von 100,0 g (0,386 mol) n-Propyl-cis-decalinylbromid (2β-Brom-6β-propyl-(4aα, 8aβ)-decahydronaphthalin) in 770 ml eines 4:1-Gemisches von Benzol und Tetrahydrofuran in der Siedehitze zugegeben. Nach beendeter Zugabe wurde weitere 30 Minuten unter Rückfluß gekocht und anschließend auf -10°C gekühlt. Nun wurde CO₂ (durch Verdampfen von Trockeneis erhalten) eingeleitet. Die Temperatur stieg auf 15°C. Nach beendeter Reaktion wurde Wasser zugegeben, mit HCl angesäuert und mit 600 ml Methyl-tert-butylether verdünnt. Die organische Phase wurde abgetrennt und einrotiert. Das erhaltene Rohprodukt wurde aus Heptan umkristallisiert. Es wurden 36,4 g (41,9 %) des Produktes als Kristalle in einer Reinheit von 99,6 % erhalten.

### Beispiel 2

### Herstellung von 2-(6β-Propyl-(4aα, 8aβ)-decahydronaphthalin-2α-yl)-1,3-dithian-2-ylium-trifluormethansulfonat.

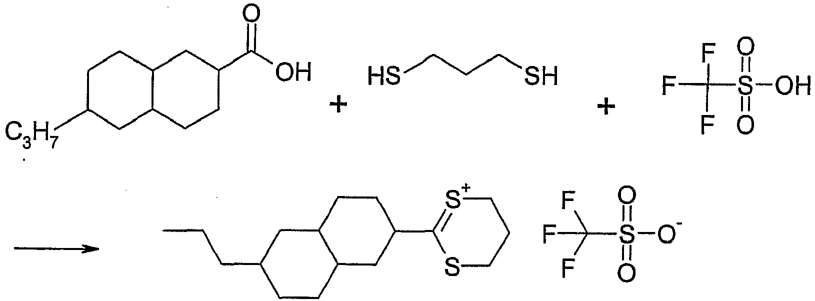

15,4 g (0,069 mol) der in Beispiel 1 erhaltenen Säure wurden mit 6,915 ml (0,069 mol) 1,3-Propandithiol und 15,6 ml (0,177 mol) Trifluormethansulfonsäure umgesetzt, indem die Säure und das Thiol vorgelegt und Trifluormethansulfonsäure zugetropft wurde. Nach Abschluß der leicht exothermen Reaktion wurde über 75 Minuten bei 120°C gerührt. Nach Abkühlen auf ca. 80°C wurden 42 ml Dibutylether zugegeben. Nach Zugabe von weiteren 100 ml Dibutylether wurde die Lösung über Nacht bei -20°C aufbewahrt. Es wurden 49,2 g eines Öls erhalten, das als solches in die nächste Stufe eingesetzt wurde. Der Gehalt an Dithianyliumsalz wurde auf 50 % geschätzt. Durch Digerieren mit Diethylether bei -80°C lassen sich 21,9 g Kristalle aus dem bei einem Wiederholungsansatz erhaltenen Öl gewinnen.

### Beispiel 3

### Herstellung von 2α-[(Difluor-3,4,5-trifluorphenoxy)-methyl]-6β-propyl-(4aα, 8aβ)-decahydronaphthalin.

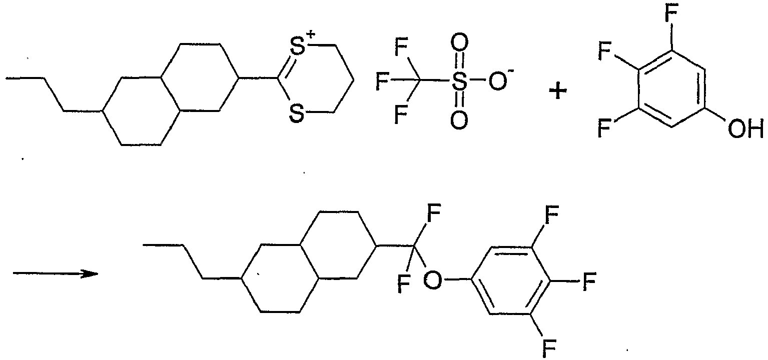

49,2 g (0,035 mol) des in Beispiel 2 erhaltenen Produkts mit einem angenommenen Gehalt von 50 % wurden in 300 ml Dichlormethan bei -70°C vorgelegt und eine Mischung aus 23,1 ml (0,166 mol) Triethylamin und 8,75 g (0,053 mol) 3,4,5-Trifluorphenol in 100 ml Dichlormethan bei dieser Temperatur zugetropft. Dabei fiel zwischenzeitlich ein farbloser Feststoff aus, der sich am Ende wieder auflöste. Man ließ 1,5 Stunden bei -70°C rühren, anschließend wurden bei dieser Temperatur 29,75 ml Triethylamintrishydrofluorid zugetropft, weitere 30 Minuten rühren gelassen, anschließend wurden 48,608 g (0,170 mol) 1,3-Dibrom-5,5-dimethylhydantoin zugegeben. Nach weiteren 1,5 Stunden ließ man den Ansatz auf 0°C erwärmen. Anschließend wurde die gelbe Suspension unter Rühren vorsichtig in NaCO₃-Lösung gegeben. Die organische Phase wurde abgetrennt und die wässrige Phase nochmals mit Dichlormethan extrahiert. Die vereinigten organischen Phasen wurden erneut mit gesättigter Natriumhydrogencarbonatlösung und Wasser gewaschen und das Lösungsmittel im Vakuum entfernt. Nach zweifachem Filtrieren über Kieselgel wurde das Produkt umkristallisiert. Es wurden 7,5 g (36,2 %) Produkt erhalten.

### Beispiel 4 (Vergleichsbeispiel)

### Herstellung von 2-[(Difluor-3,4,5-trifluorphenoxy)-methyl]-6-ethylnaphthalin.

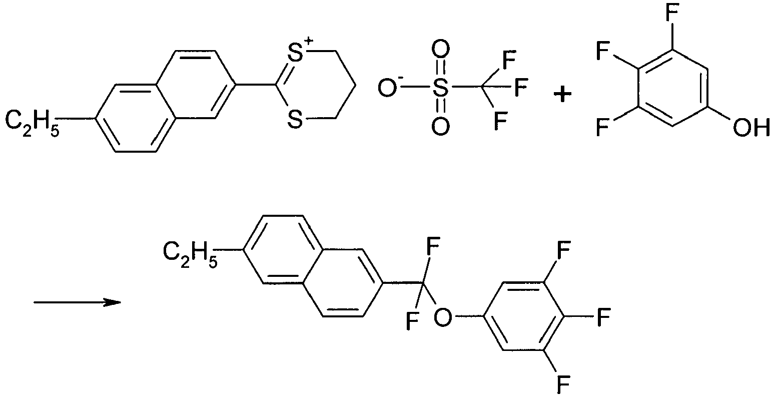

22,5 g (0,053 mol) 2-(6-Ethylnaphth-2-yl)-1,3-dithian-2-ylium-trifluormethansulfonat wurden in 440 ml Dichlormethan bei -70°C vorgelegt und eine Mischung aus 12,689 ml (0,090 mol) Triethylamin und 7,85 g (0,053 mol) 3,4,5-Trifluorphenol in 640 ml Dichlormethan bei dieser Temperatur zugetropft. Dabei fiel zwischenzeitlich ein farbloser Feststoff aus, der sich am Ende wieder auflöste. Man ließ 1,5 Stunden bei -70°C rühren, anschließend wurden bei dieser Temperatur 44,942 ml Triethylamintrishydrofluorid zugetropft, weitere 30 Minuten Rühren gelassen, anschließend wurden 73,65 g (0,257 mol) 1,3-Dibrom-5,5-dimethylhydantoin zugegeben. Nach weiteren 1,5 Stunden ließ man den Ansatz auf 0°C erwärmen. Anschließend wurde die gelbe Suspension unter Rühren vorsichtig in NaHCO₃-Lösung gegeben. Die organische Phase wurde abgetrennt und die wässrige Phase nochmals mit Dichlormethan extrahiert. Die vereinigten organischen Phasen wurden erneut mit gesättigter Natriumhydrogencarbonatlösung und Wasser gewaschen und das Lösungsmittel im Vakuum entfernt. Nach Filtrieren über Kieselgel und einer Chromatographie über Kieselgel mit Heptan/Toluol (9:1) wurde das Produkt umkristallisiert. Es wurden 1,9 g (10,1 %) Produkt erhalten.

Das Edukt, 2-(6-Ethylnaphth-2-yl)-1,3-dithian-2-ylium-trifluormethansulfonat wurde aus 6-Ethylnaphthalin-2-carbonsäure auf analoge Weise, wie in den Beispielen 1 und 2 beschrieben, erhalten.

### Beispiel 5 (Vergleichsbeispiel)

### Herstellung von 2-[(Difluor-(3,5-difluor-4-trifluormethoxy)-phenoxy)-methyl]-6-ethylnaphthalin.

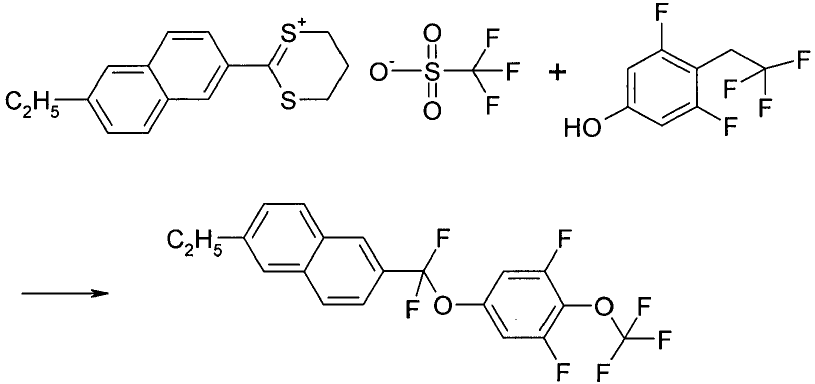

22,5 g (0,053 mol) 2-(6-Ethylnaphth-2-yl)-1,3-dithian-2-ylium-trifluormethansulfonat wurden in 440 ml Dichlormethan bei -70°C vorgelegt und eine Mischung aus 12,69 ml (0,090 mol) Triethylamin und 13,349 g (0,053 mol) 3,5-Difluor-4-(trifluormethoxy)-phenol in 200 ml Dichlormethan bei dieser Temperatur zugetropft. Dabei fiel zwischenzeitlich ein farbloser Feststoff aus, der sich am Ende wieder auflöste. Man ließ 1,5 Stunden bei -70°C rühren, anschließend wurden bei dieser Temperatur 44,942 ml Triethylamintrishydrofluorid zugetropft, weitere 30 Minuten Rühren gelassen, anschließend wurden 73,65 g (0,257 mol) 1,3-Dibrom-5,5-dimethyl-hydantoin zugegeben. Nach weiteren 1,5 Stunden ließ man den Ansatz auf 0°C erwärmen. Anschließend wurde die gelbe Suspension unter Rühren vorsichtig in NaHCO₃-Lösung gegeben. Die organische Phase wurde abgetrennt und die wässrige Phase nochmals mit Dichlormethan extrahiert. Die vereinigten organischen Phasen wurden erneut mit gesättigter Natriumhydrogencarbonatlösung und Wasser gewaschen und das Lösungsmittel im Vakuum entfernt. Nach Filtrieren über Kieselgel wurde das Produkt umkristallisiert. Es wurden 5,2 g (23,3 %) Produkt erhalten.

### Beispiel 6 (Vergleichsbeispiel)

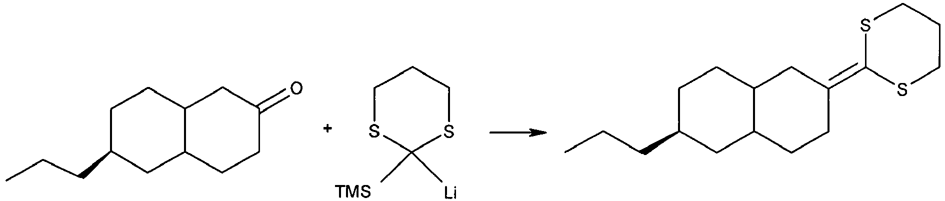

50,0 g (0,260 mol) 2-Trimethylsilyl-1,3-dithian wurden in 900 ml THF gelöst und bei -70°C 167 ml (0,273 mol) einer 15 %-igen Lösung von n-Butyllithium in Hexan zutropfen gelassen. Man ließ den Ansatz innerhalb von 4 Stunden allmählich auf 0°C auftauen, kühlte erneut auf -70°C und ließ anschließend eine Lösung von 50,0 g (0,260 mol) 6-n-Propyl-trans-decalin-2-on in 100 ml THF zutropfen. Nach beendeter Zugabe wurde die Kühlung entfernt und man ließ die klare gelbe Lösung über Nacht rühren. Anschließend gab man den Ansatz auf 1000 ml Eiswasser, trennte die wäßrige Phase ab und extrahierte diese dreimal mit je 300 ml Petrolether. Die vereinigten organischen Phasen wurden zweimal mit gesättigter Natriumchloridlösung gewaschen und über Natriumsulfat getrocknet. Das Lösungsmittel wurde im Vakuum entfernt und das Rohprodukt aus n-Heptan umkristallisiert. Man erhielt 63,2 g (82 %) des 2-(6β-Propyl-(4aα, 8aβ)-decahydronaphth-2-yliden-1,3-dithians als farblosen Feststoff.

### Beispiel 7 (Vergleichsbeispiel)

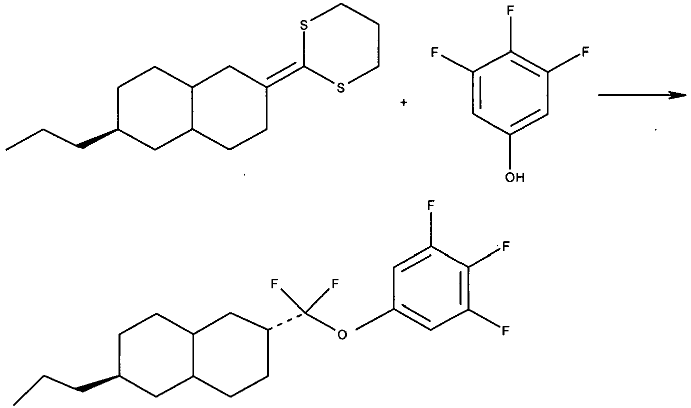

50,0 g (0,169 mol) 2-(6β-Propyl-(4aα, 8aß)-decahydronaphth-2-yliden-1,3-dithian wurden in 200 ml Dichlormethan gelöst und unter Eiskühlung 14,8 ml (0,169 mol) Trifluormethansulfonsäure vorsichtig hinzugefügt. Nach 15 Minuten wurde die Kühlung entfernt und noch 30 Minuten bei Raumtemperatur gerührt. Anschließend wurde der Ansatz auf -70°C gekühlt, eine Mischung aus 42,3 ml (0,304 mol) Triethylamin und 37,5 g (0,254 mol) 3,4,5-Trifluorphenol in 100 ml Dichlormethan hinzugegeben und 1 Stunde bei -70°C gerührt. Dann wurde die Lösung mit 136 ml (0,845 mol) Triethylamintrishydrofluorid versetzt und nach 5 Minuten eine Suspension von 242 g (0,845 mol) 1,3-Dibrom-5,5-dimethylhydantoin in 300 ml Dichlormethan innerhalb von 30 Minuten portionsweise hinzugegeben. Man ließ noch 60 Minuten rühren, ließ den Ansatz auf -20°C auftauen und gab die orangefarbene Lösung unter Rühren auf 1 l eiskalte 1 M Natronlauge. Die organische Phase wurde abgetrennt und die wäßrige Phase dreimal mit Dichlormethan extrahiert. Die vereinigten organischen Phasen wurden zweimal mit gesättigter Natriumchloridlösung gewaschen und über Natriumsulfat getrocknet. Nach Entfernen des Lösungsmittels im Vakuum wurde der Rückstand mit n-Hexan über Kieselgel filtriert und das Rohprodukt aus n-Hexan umkristallisiert. Man erhielt 55,3 g (87 %) des 2α-[(Difluor-3,4,5-trifluorphenoxy)-methyl]-6β-propyl-(4aα, 8aβ)-decahydronaphthalin als farblose Kristalle (Schmelzpunkt: 56°C).

## Patentansprüche

1. Verfahren zur Herstellung einer Verbindung der allgemeinen Formel
R-(A¹-Z-)ₘ B-CF₂O-A²(A³)ₙ-R' (I)
in der
R Alkyl, in dem eine oder mehrere CH₂-Gruppen unabhängig voneinander durch O, CF₂, CH=CH, CH=CF, CF=CF ersetzt sein können mit der Maßgabe, dass Peroxidstrukturen O-O und Formaldehyd-acetale O-CH₂-O ausgeschlossen sind,
A¹ unabhängig voneinander 1,4-Cyclohexylen, 2,5-1,3-Dioxanylen, 1,3-Cyclobutylen oder 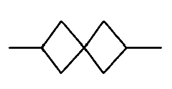
A², A³ 1,4-Phenylen, in denen unabhängig voneinander ein bis vier Wasserstoffe durch Fluor oder ein oder zwei CH-Gruppen durch N ersetzt sein können,
Z Einfachbindung, -CH₂-CH₂-, -CF₂-CF₂-, -CH=CH-, -CF=CF-, -CH=CF- oder -CF=CH-,
B 2,6-disubstituiertes trans-Dekalin,
R' R, F, OCF₃, OCF₂H, CF₃, Cl, SF₅, CN, NCS, und
m, n unabhängig voneinander 0 oder 1 bedeuten,
umfassend die folgenden Schritte:
a) Überführen einer Verbindung der allgemeinen Formel
R-(A¹-Z-)ₘBX (II),
in der X ein Halogen bedeutet und die anderen Symbole die in Bezug auf Formel (I) angegebene Bedeutung haben, in die entsprechende Grignard-Verbindung, Umsetzen der erhaltenen Grignard-Verbindungen mit CO₂ und Hydrolyse zur entsprechenden Carbonsäure der Formel
R-(A¹-Z-)ₘB-CO₂H (IV),
b) Überführung der Carbonsäure der Formel (IV) in das entsprechende Bisalkylthionium-Salz und Umsetzung dieses Salzes mit einem Phenol der allgemeinen Formel
HO-A²(-A³)ₙ-R' (III),
in der A², A³, R' und n die in Bezug auf Formel (I) angegebene Bedeutung haben, unter wasserabspaltenden Bedingungen und unter Erhalt der entsprechenden Ester, und
c) Umsetzung der erhaltenen Ester in einer oxidativen Fluordesulfurierung in die entsprechenden Ether der allgemeinen Formel (I).

2. Verfahren nach Anspruch 1, **dadurch gekennzeichnet, dass** X in Formel (II) ausgewählt ist aus der Gruppe Cl, Br und I.

3. Verfahren nach Anspruch 1 oder 2, **dadurch gekennzeichnet, dass** zur Herstellung der Grignard-Verbindung eine Verbindung der Formel (II) mit B = 2,6-Dekalinyl mit Magnesium in einem Lösungsmittel, das mindestens ein unpolares Lösungsmittel und mindestens ein polares Lösungsmittel aufweist, unter externer Wärmezufuhr umgesetzt wird.

4. Verfahren nach einem der Ansprüche 1 bis 3, **dadurch gekennzeichnet, dass** die Umsetzung der Grignard-Verbindung mit gasförmigem CO₂ erfolgt.

5. Verfahren nach Anspruch 1, **dadurch gekennzeichnet, dass** die Carbonsäure (IV) mit einem Alkylthiol zum Bisalkylthionium-Salz umgesetzt wird.

6. Verfahren nach Anspruch 5, **dadurch gekennzeichnet, dass** das Alkylthiol ein cyclisches Alkylthiol ist.

7. Verfahren nach Anspruch 5 oder 6, **dadurch gekennzeichnet, dass** das Alkylthiol ausgewählt ist aus Ethandithiol, Propandithiol und 1,2-Benzoldithiol.

8. Verfahren nach einem der Ansprüche 1 bis 7, **dadurch gekennzeichnet, dass** bei der Fluordesulfurierung als Oxidationsmittel eine Verbindung eingesetzt wird, die Haloniumäquivalente freisetzt.

9. Verfahren nach Anspruch 8, **dadurch gekennzeichnet, dass** das Oxidationsmittel ausgewählt ist aus Dibromhydantoin, Dimethyldibromhydantoin, N-Bromsuccinimid, N-Iodsuccinimid, 1,3-Dibrom-5,5-Dimethylhydantoin, SO₂Cl₂, SO₂ClF, Nitrosonium- und Nitroniumsalze, Chloramin T und Brom.

10. Verfahren nach einem der Ansprüche 1 bis 9, **dadurch gekennzeichnet, dass** das Fluorierungsmittel ausgewählt ist aus aliphatischen und aromatischen Amin-Fluorwasserstoff-Komplexen, Pyridin-Fluorwasserstoffkomplexen, NEt₃•3HF, 50 % HF in Pyridin, Melamin•HF und Polyvinylpyridin•HF.

11. Verfahren nach einem der Ansprüche 1 bis 10, **dadurch gekennzeichnet, dass** der Ester unter Bildung der Thioester als Zwischenprodukt mit einem Fluorierungsmittel in Gegenwart eines Oxidationsmittels zu den Ethern (I) umgesetzt wird.

## Claims

1. Process for the preparation of a compound of the general formula
R-(A¹-Z-)m B-CF₂O-A²-(A³)ₙ-R' (I)
in which
R denotes alkyl, in which one or more CH₂ groups may be replaced, independently of one another, by O, CF₂, CH=CH, CH=CF, CF=CF, with the proviso that peroxide structures O-O and formaldehyde acetals O-CH₂-O are excluded,
A¹, independently of one another, denotes 1,4-cyclohexylene, 2,5-1,3-dioxanylene, 1,3-cyclobutylene or 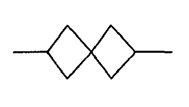
A², A³ denote 1,4-phenylene, in which, independently of one another, one to four hydrogens may be replaced by fluorine or one or two CH groups may be replaced by N,
Z denotes a single bond, -CH₂-CH₂-, -CF₂-CF₂-, -CH=CH-, -CF=CF-, -CH=CF- or -CF=CH-,
B denotes 2,6-disubstituted trans-decalin,
R' denotes R, F, OCF₃, OCF₂H, CF₃, Cl, SF₅, CN, NCS, and
m, n, independently of one another, denote 0 or 1,
comprising the following steps:
a) conversion of a compound of the general formula
R-(A¹-Z-)ₘBX (II),
in which X denotes a halogen and the other symbols have the meaning indicated in relation to formula (I), into the corresponding Grignard compound, reaction of the resultant Grignard compound with CO₂ and hydrolysis to give the corresponding carboxylic acid of the formula
R-(A¹-Z-)ₘB-CO₂H (IV),
b) conversion of the carboxylic acid of the formula (IV) into the corresponding bisalkylthionium salt and reaction of this salt with a phenol of the general formula
HO-A²(-A³)ₙ-R' (III),
in which A², A³, R' and n have the meaning indicated in relation to formula (I), under water-eliminating conditions to give the corresponding ester, and
c) conversion of the ester obtained into the corresponding ether of the general formula (I) in an oxidative fluorodesulfuration.

2. Process according to Claim 1, **characterised in that** X in formula (II) is selected from the group Cl, Br and I.

3. Process according to Claim 1 or 2, **characterised in that**, for the preparation of the Grignard compound, a compound of the formula (II) where B = 2,6-decalinyl is reacted with magnesium in a solvent which comprises at least one nonpolar solvent and at least one polar solvent, with external supply of heat.

4. Process according to one of Claims 1 to 3, **characterised in that** the reaction of the Grignard compound is carried out with gaseous CO₂.

5. Process according to Claim 1, **characterised in that** the carboxylic acid (IV) is reacted with an alkylthiol to give the bisalkylthionium salt.

6. Process according to Claim 5, **characterised in that** the alkylthiol is a cyclic alkylthiol.

7. Process according to Claim 5 or 6, **characterised in that** the alkylthiol is selected from ethanedithiol, propanedithiol and 1,2-benzenedithiol.

8. Process according to one of Claims 1 to 7, **characterised in that** the oxidant employed in the fluorodesulfuration is a compound which liberates halonium equivalents.

9. Process according to Claim 8, **characterised in that** the oxidant is selected from dibromohydantoin, dimethyldibromohydantoin, N-bromosuccinimide, N-iodosuccinimide, 1,3-dibromo-5,5-dimethyl-hydantoin, SO₂Cl₂, SO₂ClF, nitrosonium and nitronium salts, chloramine T and bromine.

10. Process according to one of Claims 1 to 9, **characterised in that** the fluorinating agent is selected from aliphatic and aromatic amine/ hydrogen fluoride complexes, pyridine/hydrogen fluoride complexes, NEt₃•3HF, 50% HF in pyridine, melamine•HF and polyvinyl-pyridine•HF.

11. Process according to one of Claims 1 to 10, **characterised in that** the ester is reacted with a fluorinating agent in the presence of an oxidant to give the ether (I) with formation of the thioester as intermediate.

## Revendications

1. Procédé de préparation d'un composé de formule générale
R-(A¹-Z-)ₘ B-CF₂O-A²-(A³)ₙ-R' (I)
dans laquelle
R désigne alkyle, où un ou plusieurs groupements CH₂ peuvent être remplacés, indépendamment les uns des autres, par O, CF₂, CH=CH, CH=CF, CF=CF, à condition que les structures de peroxyde O-O et les acétals de formaldéhyde O-CH₂-O soient exclus,
A¹, indépendamment les uns des autres, désigne 1,4-cyclohexylène, 2,5-1,3-dioxanylène, 1,3-cyclobutylène ou 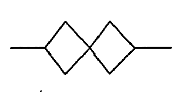
A², A³ désignent 1,4-phénylène, où, indépendamment les uns des autres, de un à quatre hydrogènes peuvent être remplacés par fluor ou un ou deux groupements CH peuvent être remplacés par N,
Z désigne une liaison simple, -CH₂-CH₂-, -CF₂-CF₂-, -CH=CH-, -CF=CF-, -CH=CF- ou -CF=CH-,
B désigne trans-décaline 2,6-disubstituée,
R' désigne R, F, OCF₃, OCF₂H, CF₃, Cl, SF₅, CN, NCS, et
m, n, indépendamment les uns des autres, désignent 0 ou 1,
comprenant les étapes suivantes :
a) la conversion d'un composé de formule générale
R-(A¹-Z-)ₘBX (II),
dans laquelle X désigne un halogène et les autres symboles revêtent la signification indiquée par rapport à la formule (I), en composé de Grignard correspondant, la réaction du composé de Grignard résultant avec du CO₂ et l'hydrolyse pour donner l'acide carboxylique correspondant de formule
R-(A¹-Z-)ₘB-CO₂H (IV),
b) la conversion de l'acide carboxylique de formule (IV) en sel de bisalkylthionium correspondant et la réaction de ce sel avec un phénol de formule générale
HO-A²(-A³)ₙ-R' (III),
dans laquelle A², A³, R' et n revêtent la signification indiquée par rapport à la formule (I), dans des conditions d'élimination d'eau pour donner l'ester correspondant, et
c) la conversion de l'ester obtenu en éther correspondant de formule générale (I) dans une fluoro-désulfuration oxydative.

2. Procédé selon la revendication 1, **caractérisé en ce que** X dans la formule (II) est choisi dans le groupe constitué par Cl, Br et I.

3. Procédé selon la revendication 1 ou 2, **caractérisé en ce que**, pour la préparation du composé de Grignard, un composé de formule (II) où B = 2,6-décalinyle est réagi avec du magnésium dans un solvant comprenant au moins un solvant non polaire et au moins un solvant polaire, avec un apport externe de chaleur.

4. Procédé selon l'une des revendications 1 à 3, **caractérisé en ce que** la réaction du composé de Grignard est effectuée avec du CO₂ gazeux.

5. Procédé selon la revendication 1, **caractérisé en ce que** l'acide carboxylique (IV) est réagi avec un alkylthiol pour donner le sel de bisalkylthionium.

6. Procédé selon la revendication 5, **caractérisé en ce que** l'alkylthiol est un alkylthiol cyclique.

7. Procédé selon la revendication 5 ou 6, **caractérisé en ce que** l'alkylthiol est choisi parmi l'éthanedithiol, le propanedithiol et le 1,2-benzènedithiol.

8. Procédé selon l'une des revendications 1 à 7, **caractérisé en ce que** l'oxydant employé dans la fluoro-désulfuration est un composé qui libère des équivalents halonium.

9. Procédé selon la revendication 8, **caractérisé en ce que** l'oxydant est choisi parmi la dibromohydantoïne, la diméthyldibromohydantoïne, le N-bromosuccinimide, le N-iodosuccinimide, la 1,3-dibromo-5,5-diméthylhydantoïne, SO₂Cl₂, SO₂ClF, les sels de nitrosonium et de nitronium, la chloramine T et le brome.

10. Procédé selon l'une des revendications 1 à 9, **caractérisé en ce que** l'agent de fluoration est choisi parmi les complexes d'amine aliphatique et aromatique/fluorure d'hydrogène, les complexes de pyridine/ fluorure d'hydrogène, NEt₃·3HF, 50% de HF dans la pyridine, la mélamine·HF et la polyvinylpyridine·HF.

11. Procédé selon l'une des revendications 1 à 10, **caractérisé en ce que** l'ester est réagi avec un agent de fluoration en présence d'un oxydant pour donner l'éther (I) avec la formation du thioester comme intermédiaire.
